# EUROPEAN PATENT SPECIFICATION

(11) **EP 2 717 789 B1**
(45) Date of publication and mention of the grant of the patent: **15.11.2017**
(21) Application number: 12727539.4
(22) Date of filing: 06.06.2012
(51) Int. Cl.: A61B 17/11

(54) **DELIVERY SYSTEM FOR MAGNETIC ANASTOMOSIS DEVICE**
ZUFÜHRSYSTEM FÜR EINE MAGNETISCHE ANASTOMOSEVORRICHTUNG
SYSTÈME DE MISE EN PLACE POUR UN DISPOSITIF D'ANASTOMOSE MAGNÉTIQUE

(30) Priority: 09.06.2011 US 201113156890
(43) Date of publication of application: 16.04.2014
(73) Proprietor: Cook Medical Technologies LLC, Bloomington, IN 47404 (US)
(72) Inventor: AGUIRRE, Andres, F., Chicago, IL (US); RUCKER, Brian, K., King, NC 27021 (US); CHMURA, Kevin, Winston-salem, NC 27023 (US)
(74) Representative: Wächter, Jochen
(86) International application number: PCT/US2012/041061
(87) International publication number: WO 2012/170502

(56) References cited:
- EP-A1- 1 493 391
- US-A1- 2006 276 825
- US-A1- 2010 256 659

## Description

### FIELD OF THE INVENTION

The present invention relates to delivery devices useful in delivering magnetic anastomosis devices.

### BACKGROUND OF THE INVENTION

Magnetic anastomosis devices (MADs) are currently used to create a channel between two viscera for the purpose of redirecting bodily fluids. For example, intestinal contents or bile may be redirected in patients who have developed an obstruction of the bowel or bile duct due to such conditions as tumor, ulcer, inflammatory strictures, or trauma. A magnetic anastomosis device is disclosed in U.S. Patent No. 5,690,656. Generally, the MAD includes first and second magnet assemblies comprising magnetic cores that are surrounded by thin metal rims. Due to the magnetic attraction between the two magnetic cores, the walls of two adjacent viscera may be sandwiched and compressed between the magnet assemblies, resulting in ischemic necrosis of the walls to produce an anastamosis between the two viscera. The viscera treated by MADs include the gall bladder, the common bile duct, the stomach, the duodenum, and the jejunum of the small intestine.

Historically, MADs have been delivered through surgical intervention such as laparotomy, which of course is invasive and carries its own risks. The exemplary self-centering MAD of U.S. Patent No. 5,690,656 permits delivery of the device over a wire guide and through the oral cavity, and typically under fluoroscopy. Alternatively, delivery can be accomplished by simply swallowing the magnet assemblies of the MAD and using massage under fluoroscopy to center the two magnet assemblies. Finally, delivery of the magnet assemblies has occasionally been performed endoscopically with grasping forceps, which can be time consuming and difficult. Removal of the MAD is typically accomplished by allowing the magnet assemblies to pass through the gastrointestinal track naturally, or more typically, with a follow-up endoscopic procedure using grasping forceps. Unfortunately, the relatively large size of the magnet assemblies can make delivery and retrieval complicated. In fact, balloon dilation of bodily lumens is often required in order to deliver the magnet assemblies to the desired location. Likewise, the size of bodily lumens is often the limiting factor in the size of the magnet assemblies that can be delivered and deployed.

Certain MAD procedures utilizing a jejunal magnet require the magnet to be passed down the esophagus to the stomach, and then through the pylorus and into the jejunum. Because of the curved nature of the passages leading to the jejunum, the magnet often becomes dislodged from the delivery system during advancement and placement thereof. Passing the jejunal magnet through the pylorus may be further complicated by patients with gastric outlet obstruction. For example, an organ anastomosing apparatus for forming inter alia a jejunum anastomosis is disclosed in EP 1 493 391 A1 and comprises a flexible guide wire, a first magnet formed in disc shape and provided with a radial through hole for receiving the guide wire, a vinculum secured to the first magnet, a second magnet provided with a through hole for receiving the vinculum, and a moving member for moving the first and second magnets.

A magnet delivery system for forming an anastomosis is further described in U.S. Patent No. 8,628,548. Said magnet delivery system comprises a wire guide, a catheter having a delivery portion for advancement into a jejunal space, and a magnet removably secured to the delivery portion of the catheter between a first and a second port within the catheter by disposing the wire guide through the first port, a lumen of the magnet and the second port.

WO 2001/008 988 further described a system for endoscopically forming an anastomosis utilizing elongate magnetic devices. Each of the elongate magnetic devices may comprise multiple magnetic elements within a housing in order to increase the flexibility needed to access the target anastomosis location.

### BRIEF SUMMARY OF THE INVENTION

Herein provided is a magnet delivery system for forming an anastomosis in a visceral space. The delivery system comprises a wire guide, a catheter, a strand and a first magnet. The catheter includes a delivery portion for advancement into a visceral space, the delivery portion having a first catheter lumen extending at least partially therethrough and slidably receiving the wire guide and the delivery portion including a distal end of the catheter. The strand is connected to the catheter, and a distal end of the strand has a connector slidably connected to the wire guide. The connector is selectively detachable from the wire guide. The first magnet defines a lumen therethrough and includes an elongate jacket enclosing two magnetic members positioned along a longitudinal axis of the elongate jacket. The first magnet is detachably connected to the delivery portion of the catheter by disposing the first wire guide through the lumen of the first magnet such that a proximal end of the first magnet is positioned distal to the distal end of the catheter, and disposing the strand alongside the first magnet and connecting the connector of the strand to the wire guide at a location distal to the proximal end of the first magnet. The strand is thereby slidably connected to the catheter or fixedly attached to the distal end of the catheter.

According to more detailed aspects, the connector includes an aperture sized to slidably receive the wire guide. The connector is preferably a loop formed by the strand, the loop defining the aperture. The loop may be formed by a distal end of the strand being folded over and connected to a distal portion of the strand, or may be formed by the strand being folded over and both ends of the strand extending proximally through the catheter. Both ends of the strand may then be accessible at a proximal end of the catheter. The connector is slidably attached to the wire guide at a point distal to the first magnet, whereby the first magnet is contained between the distal end of the catheter and the connector. In one embodiment, the first magnet defines an interior opening between its proximal and distal ends, the interior opening in communication with the lumen, and wherein the connector is slidably attached to the wire guide at a point within the interior opening. When the strand is slidably connected to the catheter, the delivery portion of the catheter preferably includes a second catheter lumen extending at least partially therethrough, the second catheter lumen slidably receiving the strand. When the strand is fixedly connected to the catheter, the strand is also preferably fixedly attached to the connector, which may comprise a short tube separately formed from the strand.

According to still further detailed aspects, the system is operable between a delivery configuration and a deployed configuration while the first magnet is in vivo, the delivery configuration including the wire guide positioned within the lumen of the first magnet such that a proximal end of the first magnet is positioned distal to the distal end of the catheter, and the strand extending alongside the first magnet and the connector slidably attached to the wire guide at a location distal to the proximal end of the first magnet, the deployed configuration including the connector being detached from the wire guide and the wire guide removed from the lumen of the first magnet. A suture lock may be positioned at a proximal end of the catheter, the suture lock operable to fix the position of the strand portions extending through the suture lock relative to the catheter. Here, a proximal portion of the catheter preferably includes a side opening, and wherein the wire guide extends through the side opening and does not extend through the suture lock.

### BRIEF DESCRIPTION OF THE DRAWINGS

The accompanying drawings incorporated in and forming a part of the specification illustrate several aspects of the present invention, and together with the description serve to explain the principles of the invention. In the drawings:
Figure 1 is a perspective view of an exemplary delivery system;
Figure 2 is an overhead view of the delivery system according to Fig. 1;
Figure 3 is a perspective view of two delivery systems with complementary jejunal magnets;
Figures 4, 5 and 6 schematically depict the use of two magnet assemblies for forming a magnetic anastamosis device;
Figure 6a is a cross-sectional view of two magnets compressing the walls of two internal bodily organs to facilitate a new anastamosis;
Figure 7 is a perspective view of an embodiment of a delivery system constructed in accordance with the teachings of the present invention;
Figure 8 is a top view of the delivery system depicted in Figure 7;
Figure 9 is a cross-sectional view, partially cut-away, of the delivery system depicted in Figure 7;
Figure 10 is a perspective view of a proximal end of the delivery system depicted in Figure 7;
Figure 11 is a top view of yet another embodiment of a delivery system constructed in accordance with the teachings of the present invention;
Figure 12 is a top view of still yet another embodiment of a delivery system constructed in accordance with the teachings of the present invention;
Figure 13 is a top view of yet another embodiment of a delivery system constructed in accordance with the teachings of the present invention;
Figure 14 is a perspective view of another embodiment of a proximal end of the delivery system depicted in Figure 10; and
Figure 15 is a perspective view of yet another embodiment of a proximal end of the delivery system depicted in Figure 10.

### DETAILED DESCRIPTION OF THE INVENTION

The term "prosthesis" means any replacement for a body part or for a function of that body part or any device that enhances or adds functionality to a physiological system.

The term "catheter" generally means a medical device comprising an elongate shaft having a lumen extending at least partially therethrough, including balloon catheters, guide catheters, delivery catheters, sheaths and cannulas. An example of a catheter includes the Cook Medical Fusion™ Biliary Dilation Catheter (FS-BDC).

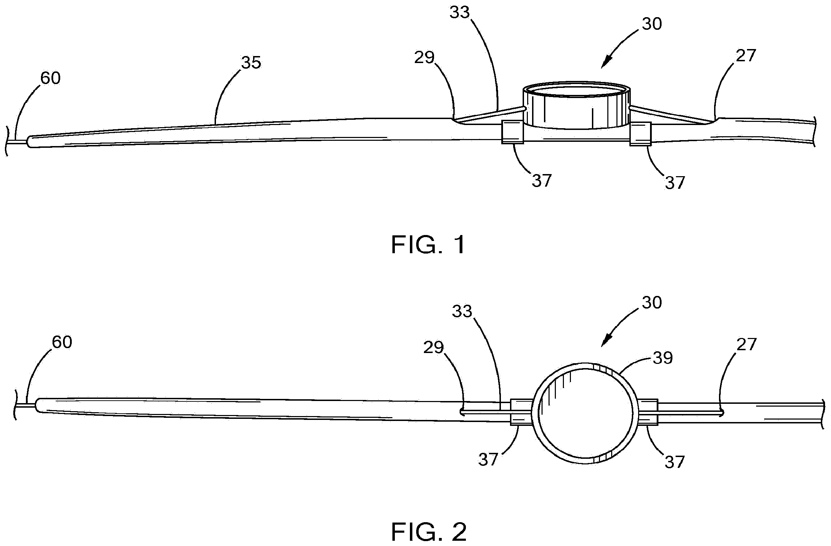

A magnet delivery system generally uses a catheter 35 and a wire guide 33 to deliver a jejunal magnet 30 as depicted in Figures 1 and 2. Suitable wire guides can include the Cook Medical Tracer Hybrid® Wire Guides (HYB-48015). In previously utilized magnet delivery systems, proximal 27 and distal 29 ports communicating with a lumen of the catheter are sufficiently spaced apart to accommodate the magnet 30 between them, wherein the wire guide 33 holds the magnet in place. Magnet sizes will range across standard sizes used in the field. These ports 27, 29 are located in the distal part of the catheter 35 and are appropriately spaced to accommodate magnets of various sizes in diameter. Magnets between about 10 mm and 20 mm in diameter or any combination or subcombination of ranges therein may be accommodated, although a magnet about 14 mm in diameter is illustrated.

The magnet 30 shown has a general disc shape (i.e. having an axial height which is less than the outer diameter). Magnets that may be used in this delivery system can be circular, cubular, cylindrical, polygonal, oval or ovoid, square, or the like. Numerous other shapes of the magnets may be readily envisioned by those skilled in the art. For example, the magnet may include an elongate magnet as described in U.S. Patent No. 8,728,105. The magnet 30 may include a protective coating which may be formed of various materials such as polymers like Teflon® or Paralene® for protection of the magnetic core from the corrosive effects of digestive acids or other bodily fluids depending upon the bodily structure involved.

The magnet 30 has a lumen therethrough to accommodate the wire guide 33. The magnet 30 also comprises an annular edge 39 along the magnet's perimeter. The edge 39 is slightly raised above the center of the magnet 30 such that it forms a basin 32 to accommodate or mate with a second magnet. In particular, when the magnet 30 is delivered, this edge 39 contacts the wall of the viscera and helps to initiate the ischemic necrosis of the tissue captured between the magnet 30 and a mated second magnet. A radiopaque marker 37 is placed on the catheter in the vicinity of the magnet to mark the magnet location when viewed through fluoroscopy. A radiopaque marker can be placed underneath the magnet 30 on the catheter 35 to mark the location of the magnet when viewing the delivery system from the side.

The catheter 35 may include radiopaque markers 37 that permit tracking of the delivery system for accurate positioning of the magnet 30. It may be preferred that a radiopaque marker 37 be placed immediately distal to the magnet 30. The catheter 35 may be used alone or in conjunction with other wire guide cannulae for navigation of the bodily lumens and delivery of a magnet.

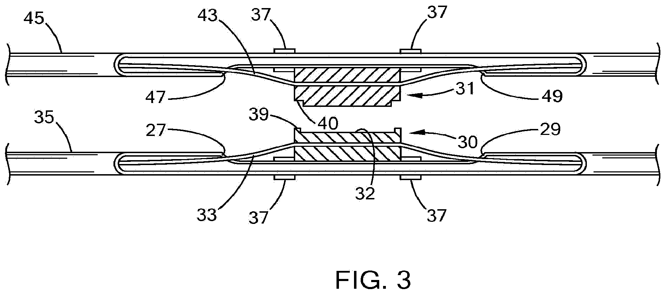

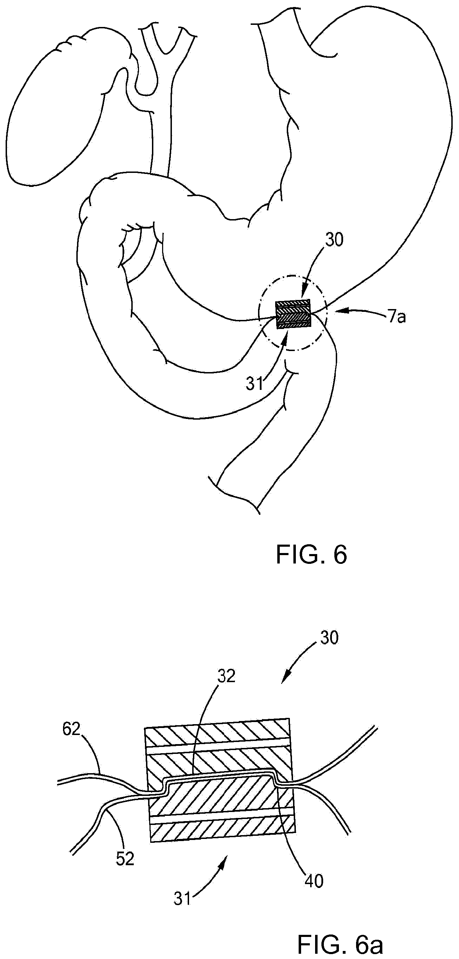

Figure 3 shows two delivery systems where a second magnet 31 is affixed to a second catheter 45. The second magnet 31 has an annular recess 40 that is capable of mating with the annular edge 39 of the first magnet 30. Figure 6a shows the walls 52, 62 of two viscera being compressed between magnets 30, 31. The edge 39 compresses the walls against the second magnet 31 to assist the ischemic necrosis. The second magnet 31 can also have an annular edge with a smaller diameter than the first magnet 30. When implanted and mated with the first magnet 30, the second magnet 31 can fit within the annular edge 39 of the first magnet 30.

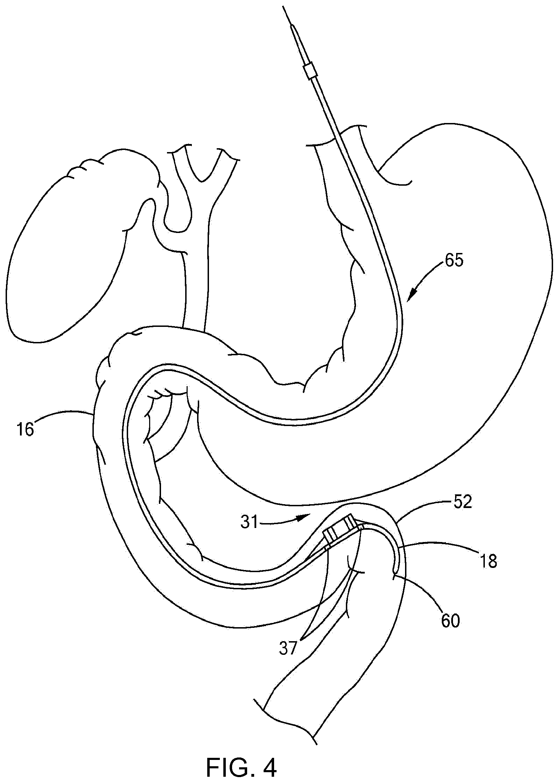

It will be recognized by those skilled in the art that the magnetic anastamosis device employing the magnet assemblies described herein not only preserves the benefits of improving the time of the procedure to place the magnet, but further provides a small delivery configuration which may be easily located within the body for accurate delivery. The delivery systems described herein also provide for insertion of the magnets through natural orifices. As such, there is also a generally applicable method for delivering the magnet assembly to a position for forming an anastamosis between two viscera. Figure 4 shows the relative positions of several viscera in the abdominal cavity, including the gall bladder 10, the common bile duct 12, the stomach 14, the duodenum 16, and the jejunum 18 of the small intestine. Although not shown, the delivery system described herein may also be used to implant anastomosis-forming magnets in the colon for possible use in gastric bypass procedures. The delivery system described herein can be used, for example, to create an anastomosis between the stomach 14 and the jejunum 18 of the small intestine. The delivery system can also be used as a part of procedure where forceps are used to place one of the magnets.

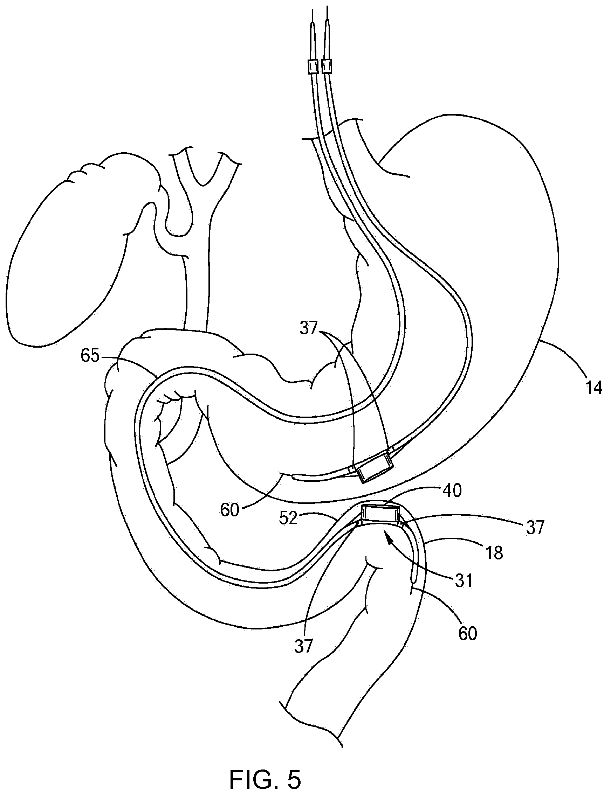

The generally applicable method for delivering a jejunal magnet to form an anastomosis comprises introducing the delivery system 65 into an endoluminal vessel. Figure 4 shows the system 65 being advanced to the jejunum 18. The delivery of magnet 31 follows once the wire guide 60 has been positioned adjacent the wall of a first viscus. In Figure 4, the first viscus is the jejunum 18. The magnet 31 is placed on catheter 35. Using the radiopaque markers 37 as a guide, the catheter 35 is advanced such that the magnet 31 is placed adjacent to the wall of the jejunum 18 as shown in Figure 5.

The delivery system 65 with magnet 31 remains in position as a second delivery system 70 is introduced into the stomach 14 as shown in Figure 5. Magnet 30 is positioned adjacent the wall of the stomach 14 that borders the jejunum 18 near the location of magnet 31. Magnets 30, 31 are released so that the magnetic forces attract the magnets together, compressing the walls 52, 62 together of the jejunum 18 and the stomach 14 as seen in Figure 6. To release magnet 31, the operator removes the wire guide 33 and then the catheter 35.

The attraction forces exerted between the magnets 30, 31 is strong enough so that in the event that the catheter 35 is caught between the two magnets 30, 31 after the placement of magnet 30, the catheter 35 may be removed and the magnets 30, 31 will remain together. The radiopaque markers 37 can be used as a guide to help position the magnet 31 in the correct orientation under fluoroscopy.

Once the necrosis of the walls of the stomach and the jejunum is complete, an anastomosis is formed. The magnets 30, 31 can then pass through the body naturally or can be removed by means such as laparotic removal, endoscopic removal, or other procedure.

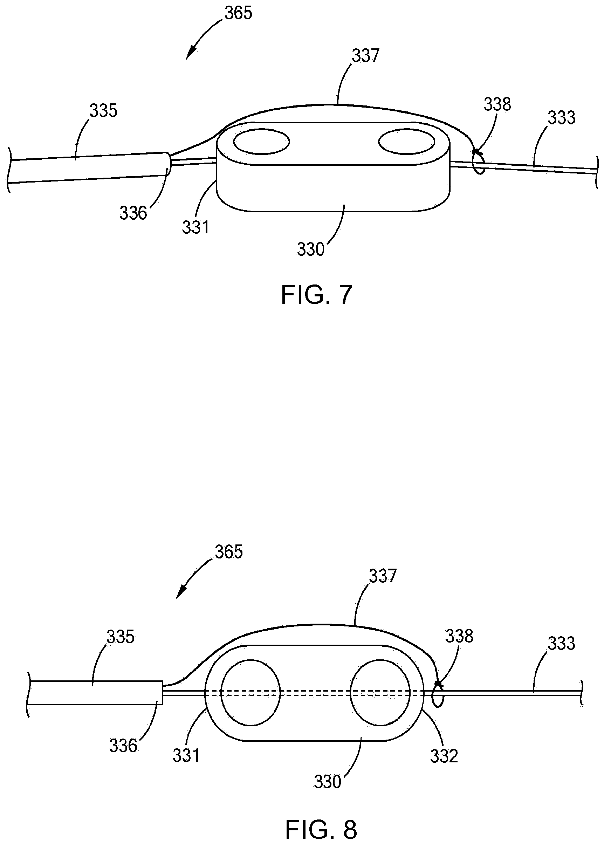

Turning now to Figures 7-10, an embodiment of a magnet delivery system 365 according to the teachings of the present invention is shown. As best seen in Figure 7, the system 365 includes a catheter 335 having a distal end 336 from which both a wire guide 333 and a strand 337 project. The strand 337 is preferably constructed of suture or other string-like material suitable for use within the body. In this embodiment, rather than being mounted directly on the catheter 335, the magnet 330 is located distally beyond the catheter 335, and in particular the proximal end 331 of the magnet 330 is positioned distal to the distal end 336 of the catheter 335. It will be recognized the magnet's proximal end 331 may abut the distal end 336 of the catheter 335. The magnet 330 includes an internal lumen 332 that slidably receives the wire guide 333. The strand 337 extends alongside and around the magnet 330, and is slidably attached to the wire guide 333 at a position distally beyond the proximal end 331 of the magnet 330, and preferably distally beyond the entire magnet 330 as shown. In the depicted embodiment, the distal end of the strand 337 is folded over and tied to itself to form a loop 338 which serves as a connector for slidably connecting the strand 337 to the wire guide.

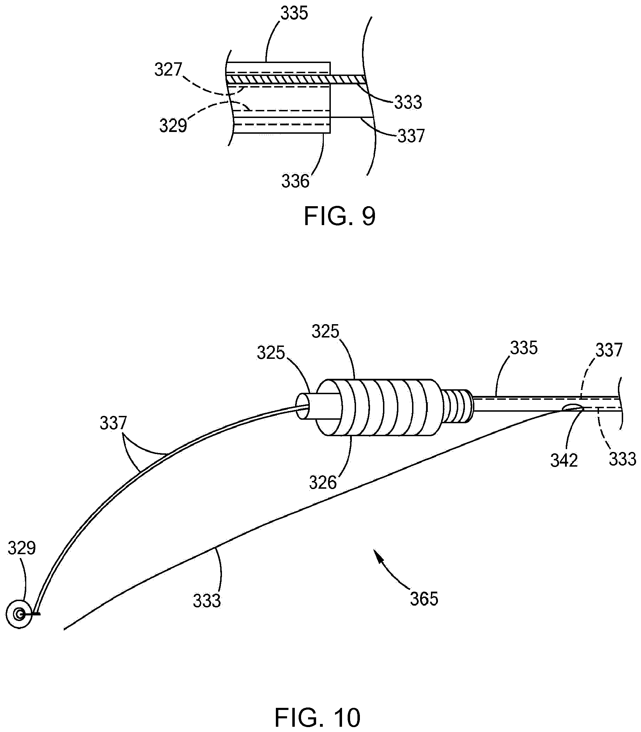

As best seen in Figure 9, the catheter 335 is preferably a dual lumen catheter, and includes a first lumen 327 and a second lumen 329 extending longitudinally through at least a portion of the catheter 335. In this manner, both the wire guide 333 and the strand 337 may move through the catheter 335 without interference with one another. As best seen in Figure 10, the proximal end of the delivery system 365 is shown, wherein the catheter 335 is connected to a proximal end device 325. The proximal end device 325 may include various known structures such as handles, visualization instruments, control instruments or the like. In the depicted embodiment, the proximal end device 325 includes a suture lock which is generally known in the art, and includes an exterior component 326 and an interior component 327 which serve to grasp and lock the strand 337 relative to the catheter 335. A ring 339 or other enlarged object may be attached to the strand 337 at its proximal end so that it does not pass completely through the suture lock 325. Preferably, the proximal end of the catheter 335 also includes a side opening 342 which is formed as a skive, and opens to only the first lumen 327 (which carries the wire guide 333). In this manner, the strand 337 may be fixed by the suture lock 325, while the wire guide 333 is allowed to bypass the lock 325 and does not interfere with it or the strand 337.

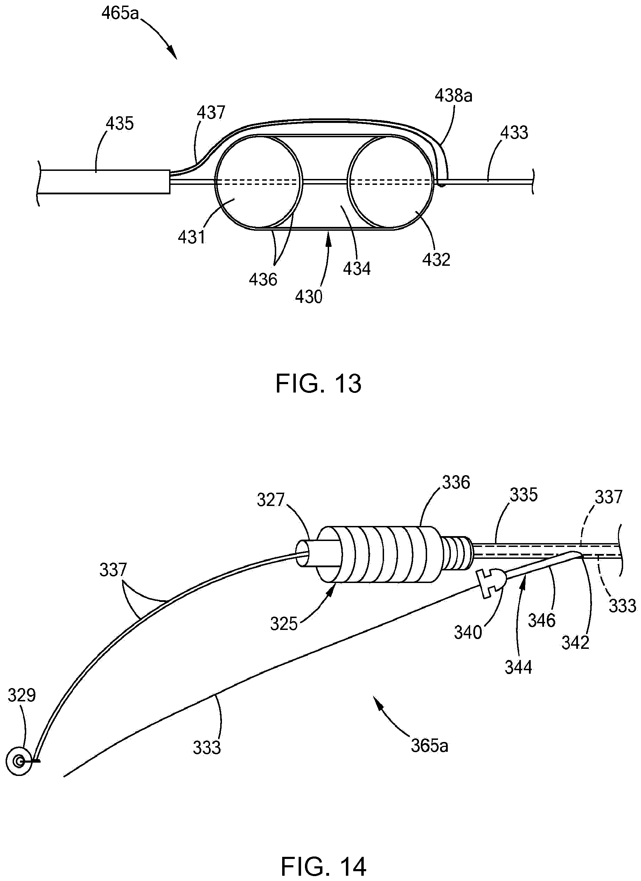

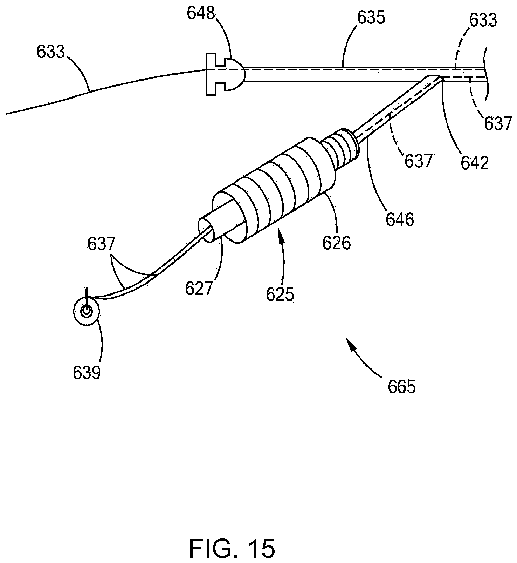

In a variation of the embodiment shown in Figure 10, the system 365a may further include a cannulated hub 344 in communication with the catheter 335 at the side opening 342 (see fig. 14). The cannulated hub 344 generally includes a short tubular member or cannula 346 having a hub 348 formed at the free, proximal, end thereof. This variation further supports the side opening 342, and the hub 348 may be designed to connect to various other catheter devices as desired. Similarly, in another variation of the system 665 shown in Figure 15, the wire guide 633 may be designed to extend linearly through the distal end of the catheter 635, while the strand 637 extends through the side opening 642. Here, the proximal end of the catheter 635 is formed with a hub 648, while the suture lock 625 (having exterior component 626 and interior component 627) is communicated to the side opening 642 via cannula 646. A ring 639 is attached to the ends of the strand 637 as in the prior embodiment.

With reference back to Figures 7 and 8, it can be seen that the tension on the strand 337 can be used to hold the magnet 330 at the distal end 336 of the catheter 335, which in turn can be fixed using the suture lock 325. In this way, the lock 325, catheter 335 and strand 337 may all be slid relative to the wire guide 333 to position the magnet 330 as discussed above. To deploy the magnet 330, the wire guide 333 may be retracted proximally such that it passes through and is removed from the connector loop 338 of the strand 337, and from the lumen 332 of the magnet 330. It will be recognized that the wire guide 333 may be withdrawn so that its distal end only passes through the connector loop 338 of the strand 337, whereby the strand 337 no longer maintains the magnet 330 at the distal end 336 of the catheter 335. The magnet 330 may then be pushed off using the catheter 335, or the wire guide 333 may simply be retracted further proximally.

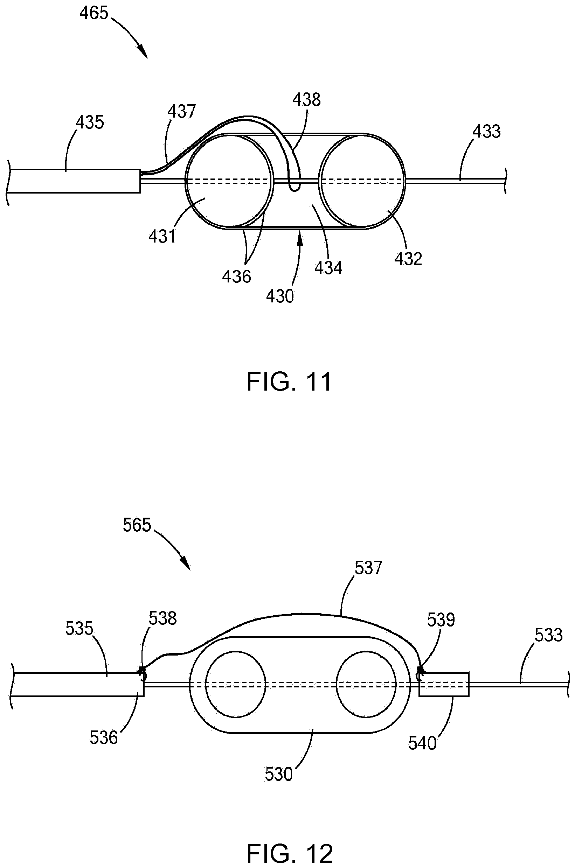

Turning now to Figure 11, another embodiment of a magnet delivery system 465 in accordance with the teachings of the present invention is shown. As with the other embodiments, the system 465 includes a catheter 435 slidably receiving a wire guide 433 and a strand 437. It can be seen that the magnet 430 includes an elongated jacket 436 or a frame 436 that supports the magnetic elements 431 and 432. The frame 436 is thus an elongated oval shape with two interior circular portions for the individual magnetic elements 431, 432. The magnet 430 and its frame 436 define an interior space 434 between the magnetic elements 431, 432 that is in communication with the lumen of the magnet 430. Accordingly, the strand 437 is permitted to pass into the interior space 434 and be slidably attached to the wire guide 433.

In the depicted embodiment, the proximal end of the strand 437 is simply wrapped around, i.e. folded over, the wire guide 433 within the interior space 434 and then extended back proximally through the same lumen of the catheter 435, leaving a loop 438 around the wire guide 433. The strand preferably extends completely to the proximal end of the system 465, such that both ends of the strand 437 are accessible and operable to the operator. In this way, the wire guide 433 does not need to be withdrawn proximally to detach it from the loop connector 438 formed by the strand 437, but rather one end of the strand 437 may be pulled from the proximal end of the system 465 to remove the loop 438 formed around the wire guide 433. As with the prior embodiments, when the wire guide 433 is completely withdrawn from the lumen of the magnet 430 the magnet 430 is deployed. It will also be recognized that, in a variation of the system 465a, the strand 437 may be wrapped around the wire guide 433 to form a loop 438a at a location distal to the magnet 430, as shown in Figure 13.

Turning now to Figure 12, yet another embodiment of a magnet delivery system 565 is shown. In this embodiment the catheter 535 includes a distal end 536 where the strand 537 is fixedly attached thereto. This may be accomplished by tying a first end 538 of the strand to the distal end 536 of the catheter 535 as shown, or it may be attached by welding, bonding, adhesives or other known attachment means. A second end 539 of the strand 537 is fixedly attached to a connector 540 by tying or any of the other above-noted means. The connector 540 is preferably a short tubular member, such as a portion of a catheter, cannula, sheath or the like. As used in describing the connector 540, the term "short" means that the tubular connector 540 has a length substantially shorter (e.g. less than 5 percent) than the length of the catheter 535. As with the prior embodiment, the wire guide 533 may be withdrawn proximally to detach it from the tubular connector 540, and then further retracted to remove it from the lumen of the magnet 530 to deploy the magnet. It will be recognized that in this embodiment, the catheter 535 need only have a single lumen (although the strand and wire guide may share a lumen in the prior embodiments), and the strand 537 is of much shorter length. Preferably the strand 537 has a length that is about one to two times the distance equal to one half of the outer perimeter of the magnet 530.

## Claims

1. A magnet delivery system (365, 365a, 465, 465a, 565, 665) for forming an anastomosis, the delivery system (365, 365a, 465, 465a, 565, 665) comprising:
a wire guide (333, 433, 533, 633);
a catheter (335, 435, 535, 635) having a delivery portion for advancement into a visceral space, the delivery portion having a first catheter lumen (327) extending at least partially therethrough and slidably receiving the wire guide (333, 433, 533, 633), the delivery portion including a distal end (336, 536) of the catheter (335, 435, 535, 635);
a strand (337, 437, 537, 637) connected to the catheter (335, 435, 535, 635), a distal end of the strand (337, 437, 537, 637) having a connector (338, 438, 540) slidably connected to the wire guide (333, 433, 533, 633), the connector (338, 438, 540) selectively detachable from the wire guide (333, 433, 533, 633); and
a first magnet (330, 430, 530) defining a lumen therethrough, wherein the first magnet (330, 430, 530) includes an elongate jacket (436) enclosing two magnetic members (431, 432) positioned along a longitudinal axis of the elongate jacket (436);
**characterized in that**
the first magnet (330, 430, 530) is detachably connected to the delivery portion of the catheter (335, 435, 535, 635) by disposing the first wire guide (333, 433, 533, 633) through the lumen of the first magnet (330, 430, 530) such that a proximal end (331) of the first magnet (330, 430, 530) is positioned distal to the distal end (336, 536) of the catheter (335, 435, 535, 635), and disposing the strand (337, 437, 537, 637) alongside the first magnet (330, 430, 530) and connecting the connector (338, 438, 540) of the strand (337, 437, 537, 637) to the wire guide (333, 433, 533, 633) at a location distal to the proximal end (331) of the first magnet (330, 430, 530); and
the strand (337, 437, 637) is slidably connected to the catheter (335, 435, 635); or
the strand (537) is fixedly attached to the distal end (536) of the catheter (535).

2. The magnet delivery system (365, 365a, 465, 465a, 565, 665) of claim 1, wherein the connector includes an aperture sized to slidably receive the wire guide (333, 433, 533, 633).

3. The magnet delivery system (365, 365a, 465, 465a, 665) of claim 2, wherein the connector is a loop (338, 438, 438a) formed by the strand (337, 437), the loop (338, 438, 438a) defining the aperture.

4. The magnet delivery system (365, 365a) of claim 3, wherein the loop (338) is formed by a distal end of the strand being folded over and connected to a distal portion of the strand (337).

5. The magnet delivery system (465, 465a) of claim 3, wherein the loop (438) is formed by the strand (437) being folded over and both ends of the strand (437) extending proximally through the catheter (435).

6. The magnet delivery system (465, 465a) of claim 5, wherein both ends of the strand (437) are accessible at a proximal end of the catheter (435).

7. The magnet delivery system (365, 465a, 565) of claim 1, wherein the connector is slidably attached to the wire guide (333, 433, 533) at a point distal to the first magnet (330, 430, 530), whereby the first magnet (330, 430, 530) is contained between the distal end (336, 536) of the catheter (335, 435, 535) and the connector.

8. The magnet delivery system (365) of claim 1, wherein the delivery portion of the catheter (335) includes a second catheter lumen (329) extending at least partially therethrough, the second catheter lumen (329) slidably receiving the strand (337).

9. The magnet delivery system (565) of claim 1, wherein the connector (540) includes a short tube separately formed from the strand (537).

10. The magnet delivery system (565) of claim 9, wherein the strand (537) is fixedly attached to the distal end (536) of the catheter (535), and wherein the strand (537) is fixedly attached to the connector (540).

11. The magnet delivery system (365, 365a, 465, 465a, 565, 665) of claim 1, wherein the system (365, 365a, 465, 465a, 565, 665) is operable between a delivery configuration and a deployed configuration while the first magnet (330, 430, 530) is in vivo, the delivery configuration including the wire guide (333, 433, 533, 633) positioned within the lumen of the first magnet (330, 430, 530) such that a proximal end (331) of the first magnet (330, 430, 530) is positioned distal to the distal end (336, 536) of the catheter (335, 435, 535, 635), and the strand (337, 437, 537, 637) extending alongside the first magnet (330, 430, 530) and the connector (338, 438, 540) slidably attached to the wire guide (333, 433, 533, 633) at a location distal to the proximal end (331) of the first magnet (330, 430, 530), the deployed configuration including the connector (338, 438, 540) being detached from the wire guide (333, 433, 533, 633) and the wire guide (333, 433, 533, 633) removed from the lumen of the first magnet (330, 430, 530).

12. The magnet delivery system (365, 365a) of claim 1, further comprising a suture lock (325) positioned at a proximal end of the catheter (335), the suture lock (325) operable to fix the position of the strand portions extending through the suture lock (325) relative to the catheter (335).

13. The magnet delivery system (365a) of claim 12, wherein a proximal portion of the catheter (335) includes a side opening (342), and wherein the wire guide (333) extends through the side opening (342) and does not extend through the suture lock (325).

## Patentansprüche

1. Magnet-Zuführanordnung (365, 365a, 465, 465a, 565, 665) zum Bilden einer Anastomose, wobei die Zuführanordnung (365, 365a, 465, 465a, 565, 665) umfasst:
einen Führungsdraht (333, 433, 533, 633);
einen Katheter (335, 435, 535, 635) mit einem Zuführabschnitt zum Fördern in einen viszeralen Raum, wobei der Zuführabschnitt einen ersten Katheter-Hohlraum (327) aufweist, der sich zumindest teilweise durch diesen hindurch erstreckt und den Führungsdraht (333, 433, 533, 633) verschiebbar aufnimmt, wobei der Zuführabschnitt ein distales Ende (336, 536) des Katheters (335, 435, 535, 635) umfasst;
einen Faden (337, 437, 537, 637), der mit dem Katheter (335, 435, 535, 635) verbunden ist, wobei ein distales Ende des Fadens (337, 437, 537, 637) ein Verbindungsglied (338, 438, 540) aufweist, das verschiebbar mit dem Führungsdraht (333, 433, 533, 633) verbunden ist, wobei das Verbindungsglied (338, 438, 540) wahlweise von dem Führungsdraht (333, 433, 533, 633) lösbar ist; und
einen ersten Magnet (330, 430, 530), der einen Hohlraum durch diesen hindurch definiert, wobei der erste Magnet (330, 430, 530) eine längliche Umhüllung (436) umfasst, die zwei magnetische Elemente (431, 432) umschließt, die entlang einer Längsachse der länglichen Umhüllung (436) angeordnet sind;
**dadurch gekennzeichnet, dass**
der erste Magnet (330, 430, 530) lösbar mit dem Zuführabschnitt des Katheters (335, 435, 535, 635) verbunden ist, indem der erste Führungsdraht (333, 433, 533, 633) derart durch den Hohlraum des ersten Magneten (330, 430, 530) angeordnet ist, dass ein proximales Ende (331) des ersten Magneten (330, 430, 530) distal des distalen Endes (336, 536) des Katheters (335, 435, 535, 635) angeordnet ist, und indem der Faden (337, 437, 537, 637) entlang des ersten Magneten (330, 430, 530) angeordnet ist und das Verbindungsglied (338, 438, 540) des Fadens (337, 437, 537, 637) mit dem Führungsdraht (333, 433, 533, 633) in einer Position distal des proximalen Endes (331) des ersten Magneten (330, 430, 530) verbunden ist; und der Faden (337, 437, 637) verschiebbar mit dem Katheter (335, 435, 635) verbunden ist; oder
der Faden (537) fest an dem distalen Ende (536) des Katheters (535) befestigt ist.

2. Magnet-Zuführanordnung (365, 365a, 465, 465a, 565, 665) nach Anspruch 1, wobei das Verbindungsglied eine Öffnung aufweist, die dimensioniert ist, um den Führungsdraht (333, 433, 533, 633) verschiebbar aufzunehmen.

3. Magnet-Zuführanordnung (365, 365a, 465, 465a, 665) nach Anspruch 2, wobei das Verbindungsglied eine Schlaufe (338, 438, 438a) ist, die durch den Faden (337, 437) gebildet ist, wobei die Schlaufe (338, 438, 438a) die Öffnung definiert.

4. Magnet-Zuführanordnung (365, 365a) nach Anspruch 3, wobei die Schlaufe (338) durch ein distales Ende des Fadens gebildet ist, das umgefaltet und mit einem distalen Abschnitt des Fadens (337) verbunden ist.

5. Magnet-Zuführanordnung (465, 465a) nach Anspruch 3, wobei die Schlaufe (438) dadurch gebildet ist, dass der Faden (437) umgefaltet wird und beide Enden des Fadens (437) sich proximal durch den Katheter (435) erstrecken.

6. Magnet-Zuführanordnung (465, 465a) nach Anspruch 5, wobei beide Enden des Fadens (437) an einem proximalen Ende des Katheters (435) zugänglich sind.

7. Magnet-Zuführanordnung (365, 465a, 565) nach Anspruch 1, wobei das Verbindungsglied in einer Position distal des ersten Magneten (330, 430, 530) verschiebbar an dem Führungsdraht (333, 433, 533) befestigt ist, wobei der erste Magnet (330, 430, 530) zwischen dem distalen Ende (336, 536) des Katheters (335, 435, 535) und dem Verbindungsglied aufgenommen ist.

8. Magnet-Zuführanordnung (365) nach Anspruch 1, wobei der Zuführabschnitt des Katheters (335) einen zweiten Katheter-Hohlraum (329) aufweist, der sich zumindest teilweise durch diesen hindurch erstreckt, wobei der zweite Katheter-Hohlraum (329) den Faden (337) verschiebbar aufnimmt.

9. Magnet-Zuführanordnung (565) nach Anspruch 1, wobei das Verbindungsglied (540) ein kurzes Röhrchen umfasst, das getrennt von dem Faden (537) ausgebildet ist.

10. Magnet-Zuführanordnung (565) nach Anspruch 9, wobei der Faden (537) fest am distalen Ende (536) des Katheters (535) befestigt ist und wobei der Faden (537) fest an dem Verbindungsglied (540) befestigt ist.

11. Magnet-Zuführanordnung (365, 365a, 465, 465a, 565, 665) nach Anspruch 1, wobei die Anordnung (365, 365a, 465, 465a, 565, 665) zwischen einem Zuführzustand und einen Einsatzzustand verstellbar ist, während der erste Magnet (330, 430, 530) in vivo ist, wobei der Zuführzustand den Führungsdraht (333, 433, 533, 633) derart innerhalb des Hohlraums des ersten Magneten (330, 430, 530) aufweist, dass ein proximales Ende (331) des ersten Magneten (330, 430, 530) distal des distalen Endes (336, 536) des Katheters (335, 435, 535, 635) angeordnet ist und sich der Faden (337, 437, 537, 637) entlang des ersten Magneten (330, 430, 530) erstreckt und das Verbindungsglied (338, 438, 540) verschiebbar in einer Position distal des proximalen Endes (331) des ersten Magneten (330, 430, 530) an dem Führungsdraht (333, 433, 533, 633) befestigt ist, wobei der Einsatzzustand das Verbindungsglied (338, 438, 540) von dem Führungsdraht (333, 433, 533, 633) gelöst aufweist und der Führungsdraht (333, 433, 533, 633) aus dem Hohlraum des ersten Magneten (330, 430, 530) entfernt ist.

12. Magnet-Zuführanordnung (365, 365a) nach Anspruch 1, weiterhin mit einer Fadenklemmung (325), die an einem proximalen Ende des Katheters (335) angeordnet ist, wobei die Fadenklemmung (325) derart bedienbar ist, dass es die Position der Fadenabschnitte, die sich durch die Fadenklemmung (325) erstrecken, relativ zum Katheter (335) festlegt.

13. Magnet-Zuführanordnung (365a) nach Anspruch 12, wobei ein proximaler Abschnitt des Katheters (335) eine seitliche Öffnung (342) aufweist und wobei sich der Führungsdraht (333) durch die seitliche Öffnung (342) erstreckt und sich nicht durch die Fadenklemmung (325) hindurch erstreckt.

## Revendications

1. Système d'administration à aimant (365, 365a, 465, 465a, 565, 665) pour former une anastomose, le système d'administration (365, 365a, 465, 465a, 565, 665) comprenant :
un guide-fil (333, 433, 533, 633) ;
un cathéter (335, 435, 535, 635) ayant une partie d'administration pour avancer dans un espace viscéral, la partie d'administration comportant une première lumière de cathéter (327) s'étendant au moins partiellement à travers cette dernière et recevant le guide-fil (333, 433, 533, 633), la partie d'administration comportant une extrémité distale (336, 536) du cathéter (335, 435, 535, 635) ;
un fil (337, 437, 537, 637) raccordé au cathéter (335, 435, 535, 635), une extrémité distale du fil (337, 437, 537, 637) ayant un connecteur (338, 438, 540) raccordé de manière coulissante au guide-fil (333, 433, 533, 633), le connecteur (338, 438, 540) pouvant être détaché de façon sélective du guide-fil (333, 433, 533, 633) ; et
un premier aimant (330, 430, 530) définissant une lumière à travers ce dernier, dans lequel le premier aimant (330, 430, 530) comprend une chemise allongée (436) renfermant deux éléments magnétiques (431, 432) positionnés autour d'un axe longitudinal de la chemise allongée (436) ;
**caractérisé en ce que**
le premier aimant (330, 430, 530) est raccordé de façon amovible à la partie d'administration du cathéter (335, 435, 535, 635) en disposant le premier guide-fil (333, 433, 533, 633) à travers la lumière du premier aimant (330, 430, 530) de telle sorte qu'une extrémité proximale (331) du premier aimant (330, 430, 530) soit positionnée de façon distale à l'extrémité distale (336, 536) du cathéter (335, 435, 535, 635) et en disposant le fil (337, 437, 537, 637) le long du premier aimant (330, 430, 530) et en raccordant le connecteur (338, 438, 540) du fil (337, 437, 537, 637) au guide-fil (333, 433, 533, 633) à une position distale à l'extrémité proximale (331) du premier aimant (330, 430, 530) ; et
le fil (337, 437, 637) étant raccordé de manière coulissante au cathéter (335, 435, 635) ; ou
le fil (537) étant raccordé à demeure à l'extrémité distale (536) du cathéter (535).

2. Système d'administration à aimant (365, 365a, 465, 465a, 565, 665) selon la revendication 1, dans lequel le connecteur comprend une ouverture dimensionnée pour recevoir de manière coulissante le guide-fil (333, 433, 533, 633).

3. Système d'administration à aimant (365, 365a, 465, 465a, 665) selon la revendication 2, dans lequel le connecteur est une boucle (338, 438, 438a) formée par le fil (337, 437), la boucle (338, 438, 438a) définissant l'ouverture.

4. Système d'administration à aimant (365, 365a) selon la revendication 3, dans lequel la boucle (338) est formée par une extrémité distale du fil qui est repliée et raccordée à une partie distale du fil (337).

5. Système d'administration à aimant (465, 465a) selon la revendication 3, dans lequel la boucle (438) est formée par le fil (437) qui est replié et les deux extrémités du fil (437) s'étendant de façon proximale à travers le cathéter (435).

6. Système d'administration à aimant (465, 465a) selon la revendication 5, dans lequel les deux extrémités du fil (437) sont accessibles au niveau d'une extrémité proximale du cathéter (435).

7. Système d'administration à aimant (365, 465a, 565) selon la revendication 1, dans lequel le connecteur est fixé de manière coulissante au guide-fil (333, 433, 533) à un point distal au premier aimant (330, 430, 530), de telle sorte que le premier aimant (330, 430, 530) soit contenu entre l'extrémité distale (336, 536) du cathéter (335, 435, 535) et le connecteur.

8. Système d'administration à aimant (365) selon la revendication 1, dans lequel la partie d'administration du cathéter (335) comprend une seconde lumière de cathéter (329) s'étendant au moins partiellement à travers cette dernière, la seconde lumière de cathéter (329) recevant de manière coulissante le fil (337).

9. Système d'administration à aimant (565) selon la revendication 1, dans lequel le connecteur (540) comprend un tube court formé de façon distincte à partir du fil (537).

10. Système d'administration à aimant (565) selon la revendication 9, dans lequel le fil (537) est fixé à demeure à l'extrémité distale (536) du cathéter (535) et dans lequel le fil (537) est fixé à demeure au connecteur (540).

11. Système d'administration à aimant (365, 365a, 465, 465a, 565, 665) selon la revendication 1, dans lequel le système (365, 365a, 465, 465a, 565, 665) est utilisable entre une configuration d'administration et une configuration déployée pendant que le premier aimant (330, 430, 530) est in vivo, la configuration d'administration comprenant le guide-fil (333, 433, 533, 633) positionné dans la lumière du premier aimant (330, 430, 530) de telle sorte qu'une extrémité proximale (331) du premier aimant (330, 430, 530) soit positionnée de façon distale à l'extrémité distale (336, 536) du cathéter (335, 435, 535, 635), et le fil (337, 437, 537, 637) s'étendant le long du premier aimant (330, 430, 530) et le connecteur (338, 438, 540) étant fixé de manière coulissante au guide-fil (333, 433, 533, 633) à une position distale à l'extrémité proximale (331) du premier aimant (330, 430, 530), la configuration déployée comprenant le connecteur (338, 438, 540) qui est détaché du guide-fil (333, 433, 533, 633) et le guide-fil (333, 433, 533, 633) étant retiré de la lumière du premier aimant (330, 430, 530).

12. Système d'administration à aimant (365, 365a) selon la revendication 1, comprenant en outre un élément de blocage de suture (325) positionné au niveau d'une extrémité proximale du cathéter (335), l'élément de blocage de suture (325) étant utilisable pour fixer la position des parties de fil s'étendant à travers l'élément de blocage de suture (325) par rapport au cathéter (335).

13. Système d'administration à aimant (365a) selon la revendication 12, dans lequel une partie proximale du cathéter (335) comprend une ouverture latérale (342) et dans lequel le guide-fil (333) s'étend à travers l'ouverture latérale (342) et ne s'étend pas à travers l'élément de blocage de suture (325).
